# EUROPÄISCHE PATENTSCHRIFT

(11) **EP 1 955 690 B1**
(45) Veröffentlichungstag und Bekanntmachung des Hinweises auf die Patenterteilung: **11.04.2018**
(21) Anmeldenummer: 08002137.1
(22) Anmeldetag: 06.02.2008
(51) Int. Cl.: A61K 8/31, A61K 8/37, A61K 8/39, A61K 8/92, A61Q 19/10

(54) **Neues Duschöl**
New shower oil
Nouvelle huile de douche

(30) Priorität: 08.02.2007 DE 102007006778
(43) Veröffentlichungstag der Anmeldung: 13.08.2008
(73) Patentinhaber: Beiersdorf AG, 20253 Hamburg (DE)
(72) Erfinder: Küther, Jörg, 25469 Halstenbek (DE); Bluschke, Thorsten, 21717 Fredenbeck-Schwinge (DE); Liste, Kathrin, 88131 Lindau (DE)

(56) Entgegenhaltungen:
- EP-A1- 0 158 108
- EP-A1- 1 704 848
- EP-A2- 0 158 249
- EP-A2- 0 557 825
- EP-A2- 1 576 948
- WO-A1-2006/111256
- DE-A1- 19 910 704
- GB-A- 2 118 961
- US-A- 3 535 427
- US-A- 4 529 605

## Beschreibung

Die vorliegende Erfindung betrifft ein bei Raumtemperatur flüssiges Duschöl. welches frei von Ethanolaminderivaten ist. Insbesondere betrifft die vorliegende Erfindung im Wesentlichen wasserfreies Duschöl, das auch bei niedrigen Temperaturen eine besonders niedrige Trübungsneigung aufweist.

Im Wesentlichen wasserfreie Duschzubereitungen mit hohem Ölgehalt sind als Duschöle im Markt sehr erfolgreich. Dabei handelt es sich um Zubereitungen, die trotz hoher Ölgehalten ein mit Duschgelen vergleichbares Schaumvermögen aufweisen und zur Reinigung des Körpers unter der Dusche geeignet sind.

Ein entscheidender Nachteil dieser Produkte aus Verbrauchersicht ist die schlechte Stabilität in der Kälte. Kommen die Produkte in Kontakt mit Kälte (Temperaturen unter 6°C) kommt es häufig zu irreversiblen Trübungen, die erste Anzeichen einer Phasentrennung darstellen.

Üblicherweise verbessert man diese Kältestabilität durch Zugabe von Derivaten des Ethanolamins. Aminderivate sind nach bisherigen Erkenntnissen in kosmetischen Formulierungen nicht mehr erwünscht, da sie Reste von freien Aminen und Nitrosaminen enthalten können.

Diese Trübung wird gut durch den Trübungsindex beschrieben. Unter dem Trübungsindex versteht man die Absorption in Prozent bei einer Wellenlänge von 600 nm, und einer Schichtdicke von 1cm, gemessen mit einem handelsüblichen UV-Vis Spectrometer bei 6°C.

Der Trübungsindex wird als Indikator für die Stabilität der Einphasigkeit nach Lagerung bei 6°C über einen Zeitraum von 3 Monaten herangezogen.

Bei herkömmlichen Duschölen, beispielsweise solchen der DE 442410 beträgt der Trübungsindex <10. Wünschenswert sind Duschöle die frei von Derivaten des Ethanolamins sind, und trotzdem einen Trübungsindex von < 10 erreichen.

EP 0 691 127 beschreiben Duschölformulierungen enthaltend Derivate des Ethanolamins.

Das Dokument DE 199 10 704 A1 offenbart Alkanolamid-freie Ölbäder, die 70 bis 30 % Öl und 30 bis 70 % eines Tensidgemisches enthalten. Die Zubereitungen zeichnen sich u.a. dadurch aus, dass sie klar sind. Es wird jedoch nicht beschrieben, dass bestimmte Substanzen besonders geeignet sind, die Klarheit der Zubereitung herbeizuführen.

Diese haben in der Regel ebenfalls einen Trübungsindex von <10. Verzichtet man auf die Derivate des Ethanolamins, kommt es oft zu Kälteinstabilitäten. Dieses zeigt sich durch einen Anstieg des Trübungsindexes auf Werte von > 30. Verwendet man beispielsweise ein handelsübliches Duschöl und wandelt dies unter Weglassen des Ethanolamins ab, so weist dieses einen Trübungsindex von ca. 40 auf und zeigt eine erhebliche Kälteinstabilität.
Ein Verzicht auf Derivate des Ethanolamins war daher bisher in tensidhaltigen Formulierungen nicht möglich. Die einzige Möglichkeit einer ethanolaminfreien Formulierung bestand lediglich im totalen Verzicht auf Tenside und unpolare Zusatzstoffe. Der entscheidende Nachteil für den Verbraucher ist die nicht vorhandene Reinigungs- und Schaumleistung.
Ein weiteres Problem bei im Wesentlichen wasserfreien Duschölen ist die Langzeitstabilität.
Unter dem Begriff langzeitstabil wird eine Einphasigkeit der Formulierung über einen Zeitraum von mindestens einem Jahr unter verschiedenen Lagerbedingungen verstanden.
Diese sind Lagerung bei Raumtemperatur, Lagerung im Licht, Lagerung bei 40°C und Lagerung bei 6°C. Wünschenswert sind Duschöle mit besonders hoher Langzeitstabilität.
Im Stand der Technik war nicht erkennbar, wie beide Mängel beseitigt werden konnten.
Es hat sich nun für den Fachmann überraschend herausgestellt, dass
ein bei Raumtemperatur flüssiges Duschöl enthaltend
- 30 bis 90 Gew.-% Öl, wobei die Öle ausgewählt werden aus der Gruppe der verzweigten und unverzweigten Kohlenwasserstoffe und -wachse, der cyclischen oder linearen Silikonölen, aus der Gruppe der gesättigten oder ungesättigten, verzweigten oder unverzweigten Alkohole und/oder aus der Gruppe der Fettsäuretriglyceride, insbesondere der Triglycerinester gesättigter und/oder ungesättigter, verzweigter und/oder unverzweigter Alkancarbonsäuren einer Kettenlänge von 8 bis 24, insbesondere 12 bis 18 C-Atomen,
- 0 bis 1 Gew.-% Wasser,
- 3 bis 50 Gew.-%, bevorzugt 4 bis 30 Gew.-%, besonders bevorzugt 5 bis 20 Gew.-% schaumbildende, öllösliche Tenside, wobei die Tenside ausgewählt werden aus der Gruppe der Alkyl- oder Hydroxyalkyl-Ammoniumsalze der Fettalkoholethersulfate und/oder der Fettalkoholetherphosphate,
- 3 bis 10 Gew.-%, bevorzugt von 3 bis 6 Gew.-% Trübungsinhibitoren, ausgewählt aus den nicht-ionischen Tensiden PEG-9 Cocoglycerid, den Estern Isopropyl Palmitat, C12-C15 Alkyl Benzoat, C12-C13 Alkyl Octanoat und dem Ether Dicaprylyl Ether aufweisend einen Trübungsindex von 1 bis 10, gemessen als Absorption in Prozent bei einer Wellenlänge von 600 nm und einer Schichtdicke von 1 cm in einem UV Vis Spektrometer bei 6°C,
dadurch gekennzeichnet, dass es frei von Ethanolaminderivaten ist, ausgewählt aus der Gruppe der Fettsäureseifen der Monoethanolamine und Diethanolamine,
den Nachteilen des Standes der Technik abhilft.
Die Trübungsinhibitoren werden aus der Gruppe PEG-9 Cocoglyceride, Dicaprylyl Ether, Isopropylpalmitate, C12-15 Alkyl Benzoate oder C12-13 Alkyl Octanoate gewählt. Diese alternativen Trübungsinhibitoren, hier insbesondere Stoffklassen wie Ethoxylate und Ester, die in wasserfreien Systemen zu diesem Zweck bisher nicht zum Einsatz gekommen sind, sind in der Lage, die Kältestabilität von wasserfreien, kosmetischen, tensidhaltigen Formulierungen im Bereich unter 6°C zu verbessern. Insbesondere lassen sich durch den Einsatz von diesen Trübungsinhibitoren kältestabile Formulierungen herstellen, die frei von Derivaten des Ethanolamins sind. Dies ist von besonderem Vorteil, weil ein Verzicht auf Derivate des Ethanolamins oft eine negative Beeinflussung der Schaummenge nach sich zieht. Es wurde festgestellt, dass durch den Einsatz der Trübungsinhibitoren dieser Mangel ausgeglichen werden konnte. Darüber hinaus wird durch den Verzicht auf Derivate des Ethanolamins eine überraschend deutliche Verbesserung der Farb- und Parfümstabilität erreicht.
Das Duschöl ist frei von Ethanolaminderivaten, gewählt aus der Gruppe der Fettsäureseifen der Monoethanolamine und Diethanolamine, besonders aus der Gruppe der Kokosfettsäurediethanolamide (Cocamide DEA), Kokosfettsäuremonoethanolamide (Cocamide MEA).
Die schaumbildenden, öllöslichen Tenside werden aus der Gruppe der Alkyl- und/oder Hydroxyalkyl-Ammoniumsalze der Fettalkoholethersulfate und /oder der Fettalkoholetherphosphate, bevorzugt Monoisopropanolaminlaurylethersulfate (MIPA-Laureth Sulfate) und/oder Triisopropanolaminlaurylethersulfate (TIPA-Laureth Sulfate) gewählt.
Von großem Vorteil ist es, wenn zusätzlich Laureth-4 enthalten ist in Konzentrationen von 2 bis 25 Gew.-%, besonders bevorzugt 4 bis 18 Gew.-%. Dadurch wird u. a. eine die Verbesserung der Öllöslichkeit des Tensidsystems erreicht.

Weiter ist es von Vorteil, wenn das Duschöl im Sinne der Erfindung frei von amphoteren Tensiden ist. Dies führt zu einer Verbesserung des Trübungsindexes der Zubereitung. Weiter ist besonders bevorzugt, wenn das erfindungsgemäße Duschöl Öle aus der Gruppe Sojaöl, Rizinusöl, Sonnenblumenöl, Weizenkeimöl, Reisöl, Diestelöl, Erdnussöl, Kokosnussöl, Macadamiaöl, Mandelöl, Nachtkerzenöl, Traubenkernöl, Paraffinöl, Dimethicone (Polydimethylsiloxane) enthält. Die Erfindung umfasst auch die Verwendung eines Duschöls als Ölbad, Duschöl, Handwaschöl oder Pflegeöl. Darüber hinaus umfasst die Erfindung auch Reinigungsartikel, die ein erfindungsgemäßes Duschöl in einem selbstauflösenden Packmittel enthalten. Dies hat den Vorteil einer vorportionierten Anwendung und daher einer erleichterten Dosierung.

Wenn sich das Packmittel in ruhendem, 38°C warmem Wasser innerhalb von 15 Minuten rücksandsfrei auflöst, wird dieses als selbstauflösend bezeichnet.

Solche Packmittel können beispielhaft in Kugel-, Beutel- oder Elypsenform vorliegen. Als Material kommen transparente Polyvinylalkoholfolien zum Einsatz, die in verschiedenen Schichtdicken angeboten werden. Dabei ist es besonders bevorzugt, wenn das selbstauflösende Packmittel ein Polyvinylalkoholfolienbeutel ist, besonders bevorzugt mit einer Folienstärke von 30 bis 90 µm, bevorzugt 40 bis 75 µm, besonders bevorzugt 50 bis 70 µm. Ein solches Packmittel weist ein gutes Auflöseverhalten im Zusammenspiel mit Dichtigkeit und Transparenz dieses Materials auf.

Beispielhaft eingesetzt wurden Solublon PVAL-Folie Typ PT der Fa. Syntana und Monosol M8630 der Fa. Monosol.

Erfindungsgemäße können Duschöle können zusätzlich öllösliche Tenside, bestimmte Ölkomponenten, Trübungsinhibitoren, Silikonöle, Wirkstoffe, Antioxidantien und/oder Farbstoffe enthalten.

Die öllöslichen Tenside werden gewählt aus der Gruppe der Alkyl- und/oder Hydroxyalkyl-Ammoniumsalze der Fettalkoholethersulfate und /oder der Fettalkoholetherphosphate. Beispielhaft verwendet wurden Monoisopropanolaminlaurylethersulfate (MIPA-Laureth Sulfate) und/oder Triisopropanolaminlaurylethersulfate (TIPA-Laureth Sulfate).

Die Ölkomponente der kosmetischen oder dermatologischen Reinigungszubereitungen - beispielsweise in Form von Duschölen - im Sinne der vorliegenden Erfindung wird gewählt aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Ricinusöl, Reisöl, Mandelöl, Palmöl, Kokosöl, Palmkernöl und dergleichen mehr.

Mineralöl ist eine flüssige Mischung aus Kohlenwasserstoffen, gewonnen aus Petrolaten. Mineralöle können durch ihre Anteile an cyclischen Alkanen (Naphthenen), aliphatischen Alkanen und aromatischen Bestandteilen unterschieden werden. Besonders geeignet sind Mineralöle mit einem Anteil an aliphatischen Alkanen von 54%-66%, einer Dichte bei 20°C zwischen 0,843 g/cm³ und 0,881 g/cm³ und einer kinetischen Viskosität bei 40°C zwischen 15 mm²/s und 72mm²/s. Besonders geeignet sind Pionier 6301S, Pionier 2076 und Pionier 2071-05 von Fa. Hansen & Rosenthal, Ondina 927, Ondina 933 CPT von Fa. Shell/DEA und Paraffinoil BF3 0025 von Fa. RA.M.Oil .

Der Trübungsinhibitor im Sinne der Erfindung wird gewählt aus der Gruppe der nichtionische Tenside, wie z. B. PEG-9 Cocoglyceride, aus der Gruppe der Ether wie z. B. Dicaprylyl Ether und aus der Gruppe der Ester wie z. B. Isopropylpalmitate, C12-15 Alkyl Benzoate oder C12-13 Alkyl Octanoate.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

In vorteilhaften Ausführungsformen der vorliegenden Erfindung enthalten die erfindungsgemäßen Reinigungszubereitungen einen oder mehrere Wirkstoffe gewählt aus der Gruppe der pflanzlichen Wirkstoffe, pflanzliche Öle, Pflanzenextrakte, Vitamine und Mineralien wie insbesondere Kamille, Aloe Vera, Aprikosenkernöl, Avocadoöl, Weizenkeimöl, Granatapfelextrakt, Seerosenextrakt, Meeresalgenextrakt, Panthenol, Niacinamid, Meersalz.

Erfindungsgemäß können als günstige Antioxidantien alle für kosmetische und/oder dermatologische Anwendungen geeigneten oder gebräuchlichen Antioxidantien verwendet werden.

Erfindungsgemäß vorteilhaft wird die erfindungsgemäße Zubereitung durch einen oder mehrere Farbstoffe eingefärbt. Die Farbstoffe können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Der Gehalt an einem oder mehreren Farbstoffen ist vorteilhaft ≤ 0,05 Gew.-%, bezogen auf das Gesamtgewicht der Zubereitung.

Zur Anwendung werden die kosmetischen und dermatologischen Zubereitungen als Duschzubereitungen in der für Kosmetika üblichen Weise auf die Haut und/oder die Haare in ausreichender Menge aufgebracht.
Als Badewannenpräparate werden diese Formulierungen in der für Badeprodukte üblichen Art und Weise, oder in Form von Einzelportionen in einem selbstauflösenden Packmittel angewendet.

Die erfindungsgemäßen Zubereitungen können vorteilhaft als Duschzubereitungen, also beispielsweise als Duschöl und dergleichen, aber auch als Badewannenpräparate - wie z. B. als Ölbäder verwendet werden.

Mineralöl ist eine flüssige Mischung aus Kohlenwasserstoffen, gewonnen aus Petrolaten. Mineralöle können durch ihre Anteile an cyclischen Alkanen (Naphthenen), aliphatischen Alkanen und aromatischen Bestandteilen unterschieden werden. Beispielhaft zum Einsatz gekommen sind Pionier 6301S, Pionier 2076 und Pionier 2071-05 von Fa. Hansen & Rosenthal, Ondina 927, Ondina 933 CPT von Fa. Shell/DEA und Paraffinoil BF3 0025 von Fa. RA.M.Oil.

Dimethicone sind Polydimethylsiloxane. Zum Einsatz gekommen ist beispielhaft DC200 Fluid 100 von Fa. Dow Corning und Baysilone-Öl M100 von Fa. Bayer.

Als Farbstoffe können sowohl wasser- sowie öllösliche Farbstoffe eingesetzt werden. Beispielhaft wurde mit folgenden Farbstoffen gearbeitet: CI 14720, CI19140, CI28440, CI 42051, CI 13015.

Als Pflanzenextrakte können alle glykolischen und ethanolischen Pflanzenextrakte eingesetzt werden. Beispielhaft wurden eingesetzt: Herbal Extract Water Lily DCS der Fa.Jan Dekker und Fruitapone Granatapfel B der Fa. Symrise.

### Beispiele

| **Rohstoff** | **1*** | **2*** | **3** | **4*** | **5*** | **6*** | **7*** |
|---|---|---|---|---|---|---|---|
| Sojaöl | 40 | 34 | 41 | 46 | 20 | 5 | |
| Pionier 6301S | | | | 5 | | | |
| Pionier 2076 | | 37 | | | | 64 | |
| Ondina 933CPT | | | | | 37 | | 20 |
| Rizinusöl | 14,5 | 2 | 15 | 14 | 10 | 2,5 | |
| Mandelöl | | | 2,5 | | | 1 | 10 |
| Reisöl | | | 10 | | 2 | | |
| Glycereth-8 Reisöl | | | | | | | 2 |
| DC200 Fluid 100 | | | 3 | | 2 | | |
| Panthenol Lsg. 75% | | | | 0,1 | 3 | | |
| C13-16 Isoparaffin | | 8 | | | 5 | 8 | |
| MIPA-Laureth Sulfat | 20,5 | 2,5 | | 15 | 4 | 5 | 15 |
| TIPA-Laureth Sulfat | | | 15 | | | | 15 |
| Ethanol | | | | | | | 0,5 |
| EDTA-Lsg. 25% | | | 1 | | | | |
| Polyglyceryl-3 Diisostearat | 3 | | | | | | |
| PEG-9 Cocoglycerid | | | | | 3 | | |
| Isopropyl Palmitat | | | 3 | | | | |
| C12-15 Alkyl Benzoat | | | | | | | 3 |
| Laureth-2 | | | | | | | 2 |
| Laureth-4 | 12,5 | 1,5 | | 15 | 2 | 3 | |
| Laureth-5 | | | | | | | 10 |
| Fruitapone Granatapfel B | | | | 0,1 | 0,7 | | |
| Parfüm | 1,5 | 3 | 2 | 2 | 3,5 | 3 | |
| Lanolin Alkohol | | 1,5 | | | 1,5 | 1 | |
| Octyldodecanol | | 5 | | | | 5 | |
| BHT | 0,05 | | | 0,05 | | | |
| Benzophenon-3 | 0,5 | 0,5 | | | | | |
| Poloxamer 101 | 2 | | 2 | 2 | | | |
| Meersalz Lsg. 30% | | | | | | | 0,1 |
| Kokossäure | | | 2 | | | | |
| Isosteareth-20 | | | | | 3 | | |
| CI 14720+CI19140+ CI28440 | | 0,01 | 0,01 | 0,0005 | | 0,005 | |
| Wasser | 0,1 | 0,2 | | 0,1 | | 0,1 | 0,2 |
| Sojaöl | ad 100 | ad 100 | ad 100 | ad 100 | ad 100 | ad 100 | ad 100 |
| Trübungsindex | 9 | | 7 | 8 | | 7 | |

| **Rohstoff** | **8*** | **9** | **10*** | **11** | **12*** | **13** | **14*** |
|---|---|---|---|---|---|---|---|
| Sojaöl | | 25,5 | 9 | 40 | 5 | 15 | 10 |
| Ondina 927 | | 20 | | | 55 | | |
| Paraffinoil BF3 0025 | 60 | | 60 | | | | |
| Pionier 2071-05 | | | | | | 30 | 40 |
| Rizinusöl | | 15 | | 20 | 2,5 | 7,5 | 5 |
| Mandelöl | | 2 | | | | 1 | |
| Reisöl | | | | | | | 0,5 |
| Baysilone-Öl M100 | | | | | | 7 | |
| C13-16 Isoparaffin | | | | | 8 | 8 | 5 |
| MIPA-Laureth Sulfat | | 17 | 10 | 17,5 | 5 | 8 | |
| TIPA-Laureth Sulfat | 12 | | | | | | 6 |
| PEG-30 Dipolyhydroxystearat | 2 | | 4 | | | | |
| PEG-40 Sorbitan Perisostearat | | 2 | | | | | |
| PEG-9 Cocoglycerid | | | | | | 5 | |
| Isopropyl Palmitat | | | | 5 | | | |
| C12-15 Alkyl Benzoat | | | 3 | | | | |
| Dicaprylyl Ether | | 5 | | | | | |
| C12-13 Alkyl Octanoat | 3 | | | | | | |
| Laureth-2 | | | 5 | | | | |
| Laureth-4 | 10 | 5 | | 15 | 5 | 7 | 4 |
| Herbal Extract Water Lily DCS | | | 0,1 | 0,1 | | | |
| Parfüm | 2 | 2 | 3 | 1,5 | 2 | 3 | 2 |
| Lanolin Alkohol | | | | | 3 | 3 | 3 |
| Octyldodecanol | | | | | 5 | 3 | 5 |
| BHT | | | | 0,05 | 0,08 | 0,06 | 0,05 |
| PEG-18 Castor Oil Dioleate | | | | | 5 | | |
| Oleth-5 | | | | | | | 3 |
| CI 42051+CI 13015 | 0,05 | | 0,01 | 0,01 | | 0,01 | 0,03 |
| Wasser | | | | 0,1 | | 0,1 | 0,1 |
| Sojaöl ad 100 | ad 100 | ad 100 | ad 100 | ad 100 | ad 100 | ad 100 | ad 100 |
| Trübungsindex | | 8 | | 6 | | 7 | 7 |

| | | | | | | | |
|---|---|---|---|---|---|---|---|
| * nicht erfindungsgemäßes Beispiel | | | | | | | |

## Patentansprüche

1. Bei Raumtemperatur flüssiges Duschöl enthaltend
• 30 bis 90 Gew.-% Öl, wobei die Öle ausgewählt werden aus der Gruppe der verzweigten und unverzweigten Kohlenwasserstoffe und -wachse, der cyclischen oder linearen Silikonölen, aus der Gruppe der gesättigten oder ungesättigten, verzweigten oder unverzweigten Alkohole und/oder aus der Gruppe der Fettsäuretriglyceride, insbesondere der Triglycerinester gesättigter und/oder ungesättigter, verzweigter und/oder unverzweigter Alkancarbonsäuren einer Kettenlänge von 8 bis 24, insbesondere 12 bis 18 C-Atomen,
• 0 bis 1 Gew.-% Wasser,
• 3 bis 50 Gew.-%, bevorzugt 4 bis 30 Gew.-%, besonders bevorzugt 5 bis 20 Gew.-% schaumbildende, öllösliche Tenside, wobei die Tenside ausgewählt werden aus der Gruppe der Alkyl- oder Hydroxyalkyl-Ammoniumsalze der Fettalkoholethersulfate und/oder der Fettalkoholetherphosphate,
• 3 bis 10 Gew.-%, bevorzugt von 3 bis 6 Gew.-% Trübungsinhibitoren, wobei die Trübungsinihibitoren ausgewählt werden aus dem nicht-ionischen Tensid PEG-9 Cocoglycerid, den Estern Isopropyl Palmitat, C12-C15 Alkyl Benzoat, C12-C13 Alkyl Octanoat und dem Ether Dicaprylyl Ether,
aufweisend einen Trübungsindex von 1 bis 10, gemessen als Absorption in Prozent bei einer Wellenlänge von 600 nm und einer Schichtdicke von 1 cm in einem UV-Vis Spektrometer bei 6°C,
**dadurch gekennzeichnet, dass** es frei von Ethanolaminderivaten ist, wobei die Etanolaminderivate ausgewählt werden aus der Gruppe der Fettsäureseifen der Monoethanolamine und Diethanolamine.

2. Duschöl nach Anspruch 1, **dadurch gekennzeichnet, dass** die schaumbildenden, öllöslichen Tenside ausgewählt werden aus der Gruppe von Monoiscpropanolaminlaurylethersulfaten (MIPA-Laureth Sulfate) und/oder Triisopropanolaminlaurylethersulfaten (TIPA-Laureth Sulfate).

3. Duschöl nach Anspruch 1 und/oder2, **dadurch gekennzeichnet, dass** es frei von amphoteren Tensiden ist.

4. Duschöl nach einem der vorangehenden Patentansprüche **dadurch gekennzeichnet, dass** die Öle aus der Gruppe Sojaöl, Rizinusöl, Sonnenblumenöl, Weizenkeimöl, Reisöl, Diestelöl, Erdnussöl, Kokosnussöl, Macadamiaöl, Mandelöl, Nachtkerzenöl und Traubenkernöl ausgewählt werden.

5. Duschöl nach einem der vorangehenden Patentansprüche **dadurch gekennzeichnet, dass** die Öle aus der Gruppe der Dimethicone (Polydimethylsiloxane) gewählt werden.

6. Reinigungsartikel umfassend eine Portionspackung in einem bei Badetemperatur selbstauflösenden Packmittel enthaltend ein Duschöl nach einem der vorangehenden Patentansprüche.

7. Reinigungsartikel nach Anspruch 6 **dadurch gekennzeichnet, dass** das selbstauflösende Packmittel ein Polyvinylalkoholfoliebeutel ist, besonders bevorzugt mit einer Folienstärke von 30 bis 90 µm, bevorzugt 40 bis 75 µm, besonders bevorzugt 50 bis 70 µm.

8. Verwendung des Duschöls gemäß den Ansprüchen 1 bis 5 als Ölbad, Handwaschöl oder Pflegeöl.

## Claims

1. Shower oil, liquid at room temperature, comprising
• 30 to 90% by weight oil, wherein the oils are selected from the group of branched and unbranched hydrocarbons and hydrocarbon waxes, cyclic or linear silicone oils, from the group of saturated or unsaturated, branched or unbranched alcohols and/or from the group of fatty acid triglycerides, particularly triglycerol esters of saturated and/or unsaturated, branched and/or unbranched alkanecarboxylic acids having a chain length of 8 to 24, particularly 12 to 18 carbon atoms,
• 0 to 1% by weight water,
• 3 to 50% by weight, preferably 4 to 30% by weight, particularly preferably 5 to 20% by weight foam-forming oil-soluble surfactants, wherein the surfactants are selected from the group of alkylammonium or hydroxyalkylammonium salts of fatty alcohol ether sulfates and/or fatty alcohol ether phosphates,
• 3 to 10% by weight, preferably 3 to 6% by weight turbidity inhibitors, wherein the turbidity inhibitors are selected from the nonionic surfactant PEG-9 cocoglyceride, the esters isopropyl palmitate, C12-C15 alkyl benzoate, C12-C13 alkyl octanoate and the ether dicaprylyl ether,
having a turbidity index of 1 to 10, measured as absorption in percent at a wavelength of 600 nm and a path length of 1 cm in a UV-Vis spectrometer at 6°C,
**characterized in that** it is free of ethanolamine derivatives, wherein the ethanolamine derivatives are selected from the group of fatty acid soaps of monoethanolamines and diethanolamines.

2. Shower oil according to Claim 1, **characterized in that** the foam-forming oil-soluble surfactants are selected from the group of monoisopropanolamine lauryl ether sulfates (MIPA-Laureth Sulfate) and/or triisopropanolamine lauryl ether sulfates (TIPA-Laureth Sulfate).

3. Shower oil according to Claim 1 and/or 2, **characterized in that** it is free of amphoteric surfactants.

4. Shower oil according to any of the preceding claims, **characterized in that** the oils are selected from the group of soya oil, castor oil, sunflower oil, wheat germ oil, rice oil, thistle oil, peanut oil, coconut oil, macadamia oil, almond oil, evening primrose oil and grape seed oil.

5. Shower oil according to any of the preceding claims, **characterized in that** the oils are selected from the group of dimethicones (polydimethylsiloxanes).

6. Cleaning article comprising a portion pack in a packing material, that is self-dissolving at bath temperature, containing a shower oil according to any of the preceding claims.

7. Cleaning article according to Claim 6, **characterized in that** the self-dissolving packing material is a polyvinyl alcohol film sachet particularly preferably having a film thickness of 30 to 90 µm, preferably 40 to 75 µm, especially preferably 50 to 70 µm.

8. Use of the shower oil according to Claims 1 to 5 as an oil bath, handwash oil or care oil.

## Revendications

1. Huile de douche liquide à température ambiante, contenant
- 30 à 90% en poids d'huile, les huiles étant choisies dans le groupe des hydrocarbures et des cires hydrocarbonées ramifié(e)s et non ramifié(e)s, des huiles de silicone cycliques ou linéaires, dans le groupe des alcools saturés ou insaturés, ramifiés ou non ramifiés et/ou dans le groupe des triglycérides d'acide gras, en particulier des esters de triglycérol d'acides alcanecarboxyliques saturés et/ou insaturés, ramifiés et/ou non ramifiés d'une longueur de chaîne de 8 à 24, en particulier de 12 à 18 atomes de carbone,
- 0 à 1% en poids d'eau,
- 3 à 50% en poids, de préférence 4 à 30% en poids, de manière particulièrement préférée 5 à 20% en poids de tensioactifs formant de la mousse, solubles dans l'huile, les tensioactifs étant choisis dans le groupe des sels d'alkylammonium ou d'hydroxyalkylammonium des éthersulfates d'alcools gras et/ou des étherphosphates d'alcools gras,
- 3 à 10% en poids, de préférence 3 à 6% en poids d'inhibiteurs de trouble, les inhibiteurs de trouble étant choisis parmi le tensioactif non ionique cocoglycéride de PEG-9, les esters palmitate d'isopropyle, benzoate de C12-C15-alkyle, octanoate de C12-C13-alkyle et l'éther dicaprylyléther,
présentant un indice de trouble de 1 à 10, mesuré comme absorption en pour cent à une longueur d'onde de 600 nm et à une épaisseur de couche de 1 cm dans un spectromètre UV-Vis à 6°C, **caractérisée en ce qu'**elle est exempte de dérivés d'éthanolamine, les dérivés d'éthanolamine étant choisis dans le groupe des savons d'acide gras des monoéthanolamines et des diéthanolamines.

2. Huile de douche selon la revendication 1, **caractérisée en ce que** les tensioactifs formant de la mousse, solubles dans l'huile sont choisis dans le groupe des lauryléthersulfates de mono-isopropanolamine (MIPA-Laureth Sulfate) et/ou des lauryléthersulfates de triisopropanolamine (TIPA-Laureth Sulfate).

3. Huile de douche selon la revendication 1 et/ou 2, **caractérisée en ce qu'**elle est exempte de tensioactifs amphotères.

4. Huile de douche selon l'une quelconque des revendications précédentes, **caractérisée en ce que** les huiles sont choisies dans le groupe formé par l'huile de soja, l'huile de ricin, l'huile de tournesol, l'huile de germes de blé, l'huile de riz, huile de carthame, l'huile d'arachide, l'huile de coco, l'huile de macadamia, l'huile d'amande, l'huile d'onagre et l'huile de pépins de raisin.

5. Huile de douche selon l'une quelconque des revendications précédentes, **caractérisée en ce que** les huiles sont choisies dans le groupe des diméthicones (polydiméthylsiloxanes).

6. Article de nettoyage comprenant un emballage portion dans un emballage autodissolvant à la température du bain, contenant une huile de douche selon l'une quelconque des revendications précédentes.

7. Article de nettoyage selon la revendication 6, **caractérisé en ce que** l'emballage autodissolvant est un sachet en feuille de poly(alcool vinylique), présentant de manière particulièrement préférée une épaisseur de feuille de 30 à 90 µm, de préférence de 40 à 75 µm, de manière particulièrement préférée de 50 à 70 µm.

8. Utilisation de l'huile de douche selon les revendications 1 à 5 comme bain d'huile, huile de lavage des mains ou huile de soin.
